# EUROPEAN PATENT SPECIFICATION

(11) **EP 1 091 774 B1**
(45) Date of publication and mention of the grant of the patent: **30.10.2002**
(21) Application number: 99931589.8
(22) Date of filing: 02.07.1999
(51) Int. Cl.: A61L 24/10, A61L 27/22, A61K 38/10, A61K 38/17, A61P 31/04

(54) **BONE CEMENT WITH ANTIMICROBIAL PEPTIDES**
KNOCHENZEMENT MIT ANTIMIKROBIELLEN PEPTIDEN
CIMENT A BASE D'OS, CONTENANT DES PEPTIDES ANTIMICROBIENS

(30) Priority: 02.07.1998 EP 98202233
(43) Date of publication of application: 18.04.2001
(73) Proprietor: Stichting Skeletal Tissue Engineering Group Amsterdam, 1081 HV Amsterdam (NL)
(72) Inventor: BURGER, Elisabeth, Henri[tte, NL-2461 GK Ter Aar (NL); VAN NIEUW AMERONGEN, Arie, NL-3621 GK Breukelen (NL); WUISMAN, Paulus, Ignatius, Jozef, Maria, NL-3991 VG Houten (NL)
(74) Representative: Wittop Koning, Tom Hugo
(86) International application number: NL9900417
(87) International publication number: WO00001427

(56) References cited:
- EP-A- 0 510 912
- WO-A-92/01462
- WO-A-94/15653
- WO-A-94/20064
- WO-A-96/39202
- WO-A-97/18827
- WO-A-99/37678
- DUCAN YU ET AL: "SELF-SETTING HYDROXYAPATITE CEMENT: A NOVEL SKELETAL DRUG-DELIVERY SYSTEM FOR ANTOBIOTICS" JOURNAL OF PHARMACEUTICAL SCIENCES, vol. 81, no. 6, 1 June 1992 (1992-06-01), pages 529-531, XP000271282 ISSN: 0022-3549

## Description

The invention relates to the use of antimicrobial peptides (AMP) in calcium phosphate bone cement and forms a system which provides for slow release of the AMP for prevention and treatment of infections of the bone (osteomyelitis) and the surrounding soft tissues.

Preventing infections of the soft tissues and the bone after operations remains a cause for concern in orthopaedic and trauma surgery. Infection of bone tissues (osteomyelitis) and/or the surrounding soft tissue is very difficult to cure and this is a reason why stringent prevention is required. At this moment granules of polymethyl methacrylate (PMMA-granules) are used for this purpose. When they are placed in the surgical wound they function as a slow release system for obtaining high local concentrations of antibiotics, while the systemic concentrations remain low. Such granules are however non-re-absorbable and an additional operation is therefore necessary. The intensive use of antibiotics in human and veterinary medicine has further resulted in large scale resistance of bacteria and fungi to antibiotics such as gentamicin. New therapies for prevention and treatment of for instance osteomyelitis are therefore urgently required.

The present invention provides for this purpose a new system for the prevention and treatment of osteomyelitis, which makes use of a re-absorbable calcium phosphate cement carrier and a new class of antibiotic agents, the so-called antimicrobial peptides (AMPs).

The AMPs used in the invention are peptides consisting of an amino acid chain which contains a domain of 10 to 25 amino acids, wherein the majority of the amino acids of the one half of the domain are positively charged amino acids and the majority of the other half of the domain are uncharged amino acids.

The structure of these peptides has a number of variations. Firstly, the domain can form an α-helix, of which at least a majority of the positions 1, 2, 5, 6, 9 (12, 13, 16, 19, 20, 23 and 24) contains a positively charged amino acid, position 8 is a positive or an uncharged amino acid and at least a majority of the positions 3, 4, 7, 10, (11, 14, 15, 17, 18, 21, 22, 25) contains an uncharged amino acid. These peptides have a lateral amphipathicity, i.e. a maximum hydrophobic moment at 100°. Stated simply, these peptides are hydrophobic on the left side and hydrophilic on the right side or vice versa. These peptides are referred to herein as "type I".

The domain can further form an α-helix, of which at least a majority of the positions 1, 2, 5, 6, 9 (12, 13, 16, 19, 20, 23 and 24) contains an uncharged amino acid, position 8 is a positive or an uncharged amino acid and at least a majority of the positions 3, 4, 7, 10, (11, 14, 15, 17, 18, 21, 22, 25) contains a positively charged amino acid. These peptides have a lateral amphipathicity, i.e. a maximum hydrophobic moment at 100°. Stated simply, these peptides are hydrophobic on the right side and hydrophilic on the left side or vice versa. These peptides are designated "type II" herein and are in principle mirror-symmetrical to type I peptides.

In addition, the domain can form an α-helix, wherein at least a majority of the positions 1 to 6 (or 7 or 8 or 9 or 10 or 11 or 12) contains an uncharged amino acid and a positively charged amino acid is found at position 7 (or 8 or 9 or 10 or 11 or 12 or 13) to 25. These peptides have a longitudinal amphipathicity, i.e. a minimum hydrophobic moment at 100°. These peptides are hydrophobic on their "top" and hydrophilic on their "bottom". Such peptides are designated "type III".

Conversely, the domain can form an α-helix, wherein at least a majority of the positions 1 to 6 (or 7 or 8 or 9 or 10 or 11 or 12) contains a positively charged amino acid and an uncharged amino acid is found at position 7 (or 8 or 9 or 10 or 11 or 12 or 13) to 25. These peptides likewise have a longitudinal amphipathicity and therefore a minimum hydrophobic moment at 100°. These peptides are hydrophobic on their "bottom" and hydrophilic on their "top". Such peptides are designated "type IV".

Finally, the domain can form a so-called β-strand and contain a positively charged amino acid on at least a majority of the positions 1, 3, 5, 7, 9 (11, 13, 15, 17, 19, 21, 23 and 25) and an uncharged amino acid on at least a majority of the positions 2, 4, 6, 8, 10, (12, 14, 16, 18, 20, 22, 24). Such a β-strand is laterally amphipathic and has a maximum hydrophobic moment at 180°. The β-strand structure is flatter than the α-helix and, stated simply, is hydrophobic on the left and hydrophilic on the right or vice versa. These are "type V" peptides.

The positively charged amino acids are preferably chosen from the group consisting of ornithine (O), lysine (K), arginine (R) and histidine (H), while the uncharged amino acids are preferably chosen from the group consisting of the aliphatic amino acids glycine (G), alanine (A), valine (V), leucine (L), isoleucine (I), the amino acids with a dipolar side chain methionine (M), asparagine (N), glutamine (Q), serine (S), threonine (T), the amino acids with an aromatic side chain phenylalanine (F), tyrosine (Y), tryptophan (W). Amino acids on the border between hydrophilic and hydrophobic can be chosen from both groups or from the remaining amino acids.

Hardly any difference in activity can in principle be detected when one of the positive amino acids and/or one of the uncharged amino acids is replaced by a random amino acid. The majority of the positively charged amino acids is therefore preferably the total number of positively charged amino acids minus 1 and the majority of the uncharged amino acids is preferably the total number of uncharged amino acids minus 1.

The domain can be a part of a larger peptide but can itself also make up the entire peptide. When the domain forms part of a larger peptide, the C-terminal and/or N-terminal amino acids which are then additionally present can be random amino acids.

The following peptides of the type I are particularly recommended:

KRLFKELKFSLRKY (peptide 3)

KRLFKELLLFSLRKY (peptide 4)

KRLFKELKKSLRKY    (peptide 5)

KRLFKELLLKSLRKY (peptide 6

OOLFOELOOSLOOY    (peptide 7)

OOLFOELLOSLOOY    (peptide 8)

KRLFKKLKFSLRKY (peptide 9)

KRLFKKLLFSLRKY    (peptide 10)

A preferred peptide of the type III has the following amino acid sequence:

LLLFLLKKRKKRKY    (peptide 11)

The peptides according to the invention can also contain further modifications. These modifications are for instance an N-terminal amide ring, for instance with acetic acid anhydride, or an alternative cleavage of the synthesis resin by which the C-terminus is modified. For this latter a replacement of the C-terminal carboxylic acid group by an amide, ester, ketone, aldehyde or alcohol group can be envisaged. Peptides with such a modification are for instance:

KRLFKELKFSLRKY-amide (peptide 12)

KRLFKELLFSLRKY-amide (peptide 13)

In addition to single peptides, oligomers can also be made. These are preferably linear oligomers of the peptides according to the invention. The coupling can be head-to-head and tail-to-tail as well as head-to-tail, either by direct synthesis or by post-synthetic enzymatic coupling. For a trans-membrane pore formation a minimum peptide length is required. Oligomers of the peptides according to the invention are double length and thereby better able in principle to span the whole phospholipid double layer of the bacterial cell membrane at one time. The activity of the peptide could hereby improve even further. In addition, extension of the peptides provides stabilisation of the helix conformation. A spacer must usually be inserted. In direct synthesis of head-to-tail coupled oligomers a spacer can be inserted to size by the use of a chain of unnatural amino acids of the correct length, for instance β-alanine, γ-amino butyric acid, ε-amino caproic acid, etc. Heterodifunctional coupling reagents, such as are commercially available for coupling peptide antigens to carrier proteins (for instance 1-ethyl-3-[3-dimethylaminopropyl]carbodiimide (EDC), m-maleimidobenzoyl)-N- hydroxysuccinimide ester (MBS), N-succinimidyl 3-[pyridyldithio]propionate (SPDD) etc.) are used to make linear oligomers with an inserted spacer. For head-to-head and tail-to-tail couplings can be used trivalent amino acids such as asparagine acid (D), glutamine acid (E), ornithine (O), lysine (K), serine (S), cysteine. Such oliqomers are for instance: KRKFHEKHHSHRGYC-CYGRHSHHKEHFKRK (peptide 14) YGRHSHHKEHFKRKC-CKRKFHEKHHSHRGY (peptide 15) αN,εN-(KRKFHEKHHSHRGY)2K-amide (peptide 16) αN,εN-(KRLFKELKFSLRKY)₂K-amide        (peptide 17) αN,εN-(KRLFKKLKFSLRKY)2K-amide (peptide 18) Peptides 14 and 15 are obtained by synthesis of peptide 2 with an additional C-terminal respectively N-terminal cysteine, whereafter the oligomer is obtained by air oxidation. Peptides 16, 17 and 18 are obtained by making use of the Multiple Antigenic Peptide (MAP) strategy, wherein a lysine having on both the α- and on the ε-amino group an Fmoc protection was used as first amino acid on the synthesis resin, whereby two identical amino acid chains (peptides 2, 3 and 9) were synthesized simultaneously on one lysine molecule.

The peptides described herein have no or hardly any haemolytic activity in physiological buffers such as PBS (phosphate-buffered saline solution). A low activity against erythrocytes of human origin is an indication of low toxicity. This selectivity is essential for the use of these peptides as antibiotics.

The peptides have a wide spectrum of antibacterial and antifungal activity, even against methycillin-resistant Staphylococcus aureus (MRSA), Pseudomonas aeruginosa (which is particularly dangerous in the case of osteomyelitis) and amphotericin-B-resistant Candida albicans.

The invention further makes use of bone material which after curing forms bone cement and wherein the AMPs are distributed homogeneously in the cured bone cement. It is biocompatible, re-absorbable and inert, and forms at body temperature. The final cement moreover has sufficient strength and stiffness to serve as bone replacement.

It has been found according to the invention that the inclusion of the AMPs in the cement does not affect the mechanical properties thereof.

In order to include the AMPs in the cement, they are dissolved in a liquid medium, preferably water, and mixed with the bone material before or after curing thereof.

A blood protein-containing solution, in particular albumin, is preferably used to hold the AMPs in solution, in order to ensure a homogeneous distribution of the AMPs in the final cured bone cement.

In a preferred embodiment bone material contains calcium phosphate. With a view to the biocompatibility this is particularly a mixture of dicalcium phosphate, tricalcium phosphate, tetracalcium phosphate and/or hydroxyl-apatite.

The invention further relates to a method of manufacturing a bone material according to the invention, wherein the bone material is cured to bone cement and wherein the AMPs are distributed homogeneously in the cured bone cement. As stated, the AMPs are dissolved in a liquid medium, preferably water, and mixed with the bone material before or after curing thereof. The AMPs are preferably mixed with the bone material after curing. A longer release period is thus provided in which the AMPs can be released to the surrounding area after arranging of the bone material. The starting point here in each case is that the AMPs are always active only where this is necessary.

The invention also relates to a device for administering bone material provided with AMPs according to the invention, wherein provision is made for at least two compartments for separately containing the bone material and AMPs, a mixing chamber for mixing the bone material and the AMPs and a spray nozzle for spraying the mixture out of the mixing chamber.

The invention will be further elucidated with reference to a discussion of a number of tests in accordance with preferred variants of the invention, wherein the procedures for manufacturing the present bone material with added AMPs will be discussed.
1. A sterile cement powder consists of a mixture of alpha-tricalcium phosphate, tetracalcium phosphate-monoxide en dicalcium phosphate dibasic in a ratio of 75:20:5, or otherwise if desired.
2. A sterile AMP solution (solution (A)) consists of 4 mM HCI in water having dissolved therein 0.1% beef or human serum albumin and AMPs in a concentration as required varying from 2x10⁻⁵% to 2%.
3. A second sterile solution (solution (B)) consists of water having dissolved therein 12% sodium succinate and 5% chondroitin succinate.
4. Solution (A) is mixed 1 to 1 with solution (B) under sterile conditions.
5. One volume part solution (A+B) is mixed with two volume parts cement powder under sterile conditions. This can take place:
   a. in a dish and mixed with a spatula, whereafter the cement paste is arranged immediately in-situ in the body of the patient and there cures;
   b. via a spray with two chambers, one of which contains the cement powder and the other solution (A+B); using the spray, powder and liquid are brought together in-situ in the body, whereafter the mixture cures at this location.
   c. in a dish, mould or container, whereafter the mixture cures outside the body and is optionally ground to a powder of the desired granule size, whereafter it is arranged in the body of the patient.
6. One volume part solution B is mixed with two volume parts cement powder under sterile conditions in a dish, mould or container, whereafter the mixture cures and is ground to a powder of the desired granule size. The cured cement is then incubated for 1 or more hours in solution A, whereafter the cement with absorbed AMPs is dried and stored in dry form until it is arranged in the body of the patient.

## Claims

1. Bone material for the prevention and treatment of osteomyelitis, which material is provided with antimicrobial peptides (AMPs) consisting of an amino acid chain which contains a domain of 10 to 25 amino acids, wherein the majority of the amino acids of the one half of the domain are positively charged amino acids and the majority of the amino acids of the other half of the domain are uncharged amino acids, which AMPs can be released to the surrounding area for a period of time and wherein the bone material forms bone cement after curing and the AMPs are distributed homogeneously in the cured bone cement.

2. Bone material as claimed in claim 1, **characterized in that** the domain forms an α-helix and at least at a majority of the positions 1, 2, 5, 6, 9 (12, 13, 16, 19, 20, 23 and 24) contains a positively charged amino acid, at position 8 a positive or an uncharged amino acid and at least at a majority of the positions 3, 4, 7, 10, (11, 14, 15, 17, 18, 21, 22, 25) contains an uncharged amino acid.

3. Bone material as claimed in claim 2, **characterized in that** the positively charged amino acids are chosen from the group consisting of ornithine (O), lysine (K), arginine (R) and histidine (H).

4. Bone material as claimed in claim 2 or 3, **characterized in that** the uncharged amino acids are chosen from the group consisting of the aliphatic amino acids glycine (G), alanine (A), valine (V), leucine (L), isoleucine (I), the amino acids with a dipolar side chain methionine (M), asparagine (N), glutamine (Q), serine (S), threonine (T), the amino acids with an aromatic side chain phenylalanine (F), tyrosine (Y), tryptophan (W).

5. Bone material as claimed in claims 2-4, **characterized in that** the majority of the positively charged amino acids is the total number of positively charged amino acids minus 1.

6. Bone material as claimed in claims 2-5, **characterized in that** the majority of the uncharged amino acids is the total number of uncharged amino acids minus 1.

7. Bone material as claimed in claims 2-6, **characterized in that** the domain makes up the entire peptide.

8. Bone material as claimed in claims 2-7, of which the domain has the following amino acid sequence:

KRLFKELKFSLRKY (peptide 3).

9. Bone material as claimed in claims 2-7, of which the domain has the following amino acid sequence:

KRLFKELLFSLRKY (peptide 4).

10. Bone material as claimed in claims 2-7, of which the domain has the following amino acid sequence:

KRLFKELKKSLRKY (peptide 5).

11. Bone material as claimed in claims 2-7, of which the domain has the following amino acid sequence:

KRLFKELLKSLRKY (peptide 6).

12. Bone material as claimed in claims 2-7, of which the domain has the following amino acid sequence:

OOLFOELOOSLOOY          peptide 7).

13. Bone material as claimed in claims 2-7, of which the domain has the following amino acid sequence:

OOLFOELLOSLOOY          (peptide 8).

14. Bone material as claimed in claims 2-7, of which the domain has the following amino acid sequence:

KRLFKKLKFSLRKY (peptide 9).

15. Bone material as claimed in claims 2-7, of which the domain has the following amino acid sequence:

KRLFKKLLFSLRKY (peptide 10).

16. Bone material as claimed in claim 1, **characterized in that** the domain forms an α-helix and at least at a majority of the positions 1 to 6 (or 7 or 8 or 9 or 10 or 11 or 12) contains an uncharged amino acid and at position 7 (or 8 or 9 or 10 or 11 or 12 or 13) to 25 a positively charged amino acid.

17. Bone material as claimed in claim 1, **characterized in that** the domain forms an α-helix and at least at a majority of the positions 1 to 6 (or 7 or 8 or 9 or 10 or 11 or 12) contains a positively charged amino acid and at position 7 (or 8 or 9 or 10 or 11 or 12 or 13) to 25 an uncharged amino acid.

18. Bone material as claimed in claim 16 or 17, **characterized in that** the positively charged amino acids are chosen from the group consisting of ornithine (O), lysine (K), arginine (R) and histidine (H).

19. Bone material as claimed in claim 16, 17 or 18, **characterized in that** the uncharged amino acids are chosen from the group consisting of the aliphatic amino acids glycine (G), alanine (A), valine (V), leucine (L), isoleucine (I), the amino acids with a dipolar side chain methionine (M), asparagine (N), glutamine (Q), serine (S), threonine (T), the amino acids with an aromatic side chain phenylalanine (F), tyrosine (Y), tryptophan (W).

20. Bone material as claimed in claims 16-19, **characterized in that** the majority of the positively charged amino acids is the total number of positively charged amino acids minus 1.

21. Bone material as claimed in claims 16-20, **characterized in that** the majority of the uncharged amino acids is the total number of uncharged amino acids minus 1.

22. Bone material as claimed in claims 16-21, **characterized in that** the domain makes up the entire peptide.

23. Bone material as claimed in claims 16 and 18-22, of which the domain has the following amino acid sequence:

LLLFLLKKRKKRKY (peptide 11).

24. Bone material as claimed in claim 1, **characterized in that** the domain forms a so-called β-strand and contains a positively charged amino acid on at least a majority of the positions 1, 3, 5, 7, 9 (11, 13, 15, 17, 19, 21, 23 and 25) and an uncharged amino acid on at least a majority of the positions 2, 4, 6, 8, 10, (12, 14, 16, 18, 20, 22, 24).

25. Bone material as claimed in claim 24, **characterized in that** the positively charged amino acids are chosen from the group consisting of ornithine (O), lysine (K), arginine (R) and histidine (H).

26. Bone material as claimed in claim 24, **characterized in that** the uncharged amino acids are chosen from the group consisting of the aliphatic amino acids glycine (G), alanine (A), valine (V), leucine (L), isoleucine (I), the amino acids with a dipolar side chain methionine (M), asparagine (N), glutamine (Q), serine (S), threonine (T), the amino acids with an aromatic side chain phenylalanine (F), tyrosine (Y), tryptophan (W).

27. Bone material as claimed in claims 24-26, **characterized in that** the majority of the positively charged amino acids is the total number of positively charged amino acids minus 1.

28. Bone material as claimed in claims 24-27, **characterized in that** the majority of the uncharged amino acids is the total number of uncharged amino acids minus 1.

29. Bone material as claimed in claims 24-28, **characterized in that** the domain makes up the entire peptide.

30. Bone material as claimed in claims 1-29, wherein the N-terminus is amidated.

31. Bone material as claimed in claims 1-30, wherein the C-terminal carboxylic acid group is replaced by an amide, ester, ketone, aldehyde or alcohol group.

32. Method of manufacturing bone material as claimed in any of the claims 1-31, wherein the bone material is cured to bone cement and wherein the AMPs are distributed homogeneously in the cured bone cement.

33. Method as claimed in claim 32, wherein the AMPs are dissolved in liquid medium, preferably water, and mixed with the bone material after curing thereof.

34. Method as claimed in claim 32 or 33, wherein the cured bone cement is formed to a granulate.

## Patentansprüche

1. Knochenmaterial zur Prävention und Behandlung von Osteomyelitis, wobei das Material mit antimikrobiellen Peptiden (AMPs) ausgestattet ist, die aus einer Aminosäurekette bestehen, die eine Domäne aus 10 bis 25 Aminosäuren enthält, worin die Mehrheit der Aminosäuren der einen Hälfte der Domäne positiv geladene Aminosäuren sind und die Mehrheit der Aminosäuren der anderen Hälfte der Domäne ungeladene Aminosäuren sind, wobei die AMPs über einen Zeitraum in den umgebenden Bereich freigesetzt werden können und wobei das Knochenmaterial nach Härtung Knochenzement bildet und die AMPs homogen im gehärteten Knochenzement verteilt sind.

2. Knochenmaterial nach Anspruch 1, **dadurch gekennzeichnet, daß** die Domäne eine α-Helix bildet und in zumindestens der Mehrheit der Positionen 1, 2, 5, 6, 9 (12, 13, 16, 19, 20, 23 und 24) eine positiv geladene Aminosäure enthält, an Position 8 eine positiv oder eine ungeladene Aminosäure enthält und in mindestens der Mehrheit der Positionen 3, 4, 7, 10 (11, 14, 15, 17, 18, 21, 22, 25) eine ungeladene Aminosäure enthält.

3. Knochenmaterial gemäß Anspruch 2, **dadurch gekennzeichnet, daß** die positiv geladenen Aminosäuren aus der Gruppe bestehend aus Ornithin (O), Lysin (K), Arginin (R) und Histidin (H) ausgewählt sind.

4. Knochenmaterial gemäß Anspruch 2 oder 3, **dadurch gekennzeichnet, daß** die ungeladenen Aminosäuren aus der Gruppe, bestehend aus den aliphatischen Aminosäuren Glycin (G), Alanin (A), Valin (V), Leucin (L), Isoleucin (I), den Aminosäuren mit einer dipolaren Seitenkette Methionin (M), Asparagin (N), Glutamin (Q), Serin (S), Threonin (T), den Aminosäuren mit einer aromatischen Seitenkette Phenylalanin (F), Tyrosin (Y), Tryptophan (W), ausgewählt sind.

5. Knochenmaterial gemäß den Ansprüchen 2 bis 4, **dadurch gekennzeichnet, daß** die Mehrheit der positiv geladenen Aminosäuren die Gesamtzahl der positiv geladenen Aminosäuren minus 1 ist.

6. Knochenmaterial gemäß den Ansprüchen 2 bis 5, **dadurch gekennzeichnet, daß** die Mehrheit der ungeladenen Aminosäuren die Gesamtzahl an ungeladenen Aminosäuren minus 1 ist.

7. Knochenmaterial gemäß den Ansprüchen 2 bis 6, **dadurch gekennzeichnet, daß** die Domäne das gesamte Peptid ausmacht.

8. Knochenmaterial gemäß den Ansprüchen 2 bis 7, dessen Domäne die folgende Aminosäuresequenz hat:

KRLFKELKFSLRKY (Peptid 3).

9. Knochenmaterial gemäß den Ansprüchen 2 bis 7, wobei die Domäne die folgende Aminosäuresequenz hat:

KRLFKELLFSLRKY (Peptid 4).

10. Knochenmaterial gemäß den Ansprüchen 2 bis 7, wobei die Domäne die folgende Aminosäuresequenz hat:

KRLFKELKKSLRKY (Peptid 5).

11. Knochenmaterial gemäß den Ansprüchen 2 bis 7, wobei die Domäne die folgende Aminosäuresequenz hat:

KRLFKELLLKSLRKY          (Peptid 6).

12. Knochenmaterial gemäß den Ansprüchen 2 bis 7, wobei die Domäne die folgende Aminosäuresequenz hat:

OOLFOELOOSLOOY          (Peptid 7).

13. Knochenmaterial gemäß den Ansprüchen 2 bis 7, wobei die Domäne die folgende Aminosäuresequenz hat:

OOLFOELLOSLOOY (Peptid 8).

14. Knochenmaterial gemäß den Ansprüchen 2 bis 7, wobei die Domäne die folgende Aminosäuresequenz hat:

KRLFKKLKFSLRKY (Peptid 9).

15. Knochenmaterial gemäß den Ansprüchen 2 bis 7, wobei die Domäne die folgende Aminosäuresequenz hat:

KRLFKKLLFSLRKY     (Peptid 10).

16. Knochenmaterial gemäß Anspruch 1, **dadurch gekennzeichnet, daß** die Domäne eine α-Helix bildet und in mindestens der Mehrheit der Positionen 1 bis 6 (oder 7 oder 8 oder 9 oder 10 oder 11 oder 12) eine ungeladene Aminosäure und in Position 7 (oder 8 oder 9 oder 10 oder 11 oder 12 oder 13) bis 25 eine positiv geladene Aminosäure enthält.

17. Knochenmaterial gemäß Anspruch 1, **dadurch gekennzeichnet, daß** die Domäne eine α-Helix bildet und in mindestens der Mehrheit der Positionen 1 bis 6 (oder 7 oder 8 oder 9 oder 10 oder 11 oder 12) eine positiv geladene Aminosäure und in Position 7 (oder 8 oder 9 oder 10 oder 11 oder 12 oder 13) bis 25 eine ungeladenen Aminosäure enthält.

18. Knochenmaterial gemäß Anspruch 16 oder 17, **dadurch gekennzeichnet, daß** die positiv geladenen Aminosäuren aus der Gruppe, bestehend aus Ornithin (O), Lysin (K), Arginin (R) und Histidin (H), ausgewählt sind.

19. Knochenmaterial gemäß Anspruch 16, 17 oder 18, **dadurch gekennzeichnet, daß** die ungeladenen Aminosäuren aus der Gruppe, bestehend aus den aliphatischen Aminosäuren Glycin (G), Alanin (A), Valin (V) , Leucin (L), Isoleucin (I), den Aminosäuren mit einer dipolaren Seitenkette Methionin (M), Asparagin (N), Glutamin (Q), Serin (S), Threonin (T), den Aminosäuren mit einer aromatischen Seitenkette Phenylalanin (F), Tyrosin (Y), Tryptophan (W), ausgewählt sind.

20. Knochenmaterial gemäß den Ansprüchen 16 bis 19, **dadurch gekennzeichnet, daß** die Mehrheit der positiv geladenen Aminosäuren die Gesamtzahl der positiv geladenen Aminosäuren minus 1 ist.

21. Knochenmaterial gemäß der Ansprüche 16 bis 20, **dadurch gekennzeichnet, daß** die Mehrheit der ungeladenen Aminosäuren die Gesamtzahl der ungeladenen Aminosäuren minus 1 ist.

22. Knochenmaterial gemäß den Ansprüchen 16 bis 21; **dadurch gekennzeichnet, daß** die Domäne das gesamte Peptid ausmacht.

23. Knochenmaterial gemäß den Ansprüchen 16 und 18 bis 22, wobei die Domäne die folgende Aminosäuresequenz hat:

LLLFLLKKRKKRKY (Peptid 11).

24. Knochenmaterial gemäß Anspruch 1, **dadurch gekennzeichnet, daß** die Domäne einen sogenannten β-Strang bildet und eine positiv geladene Aminosäure mindestens in der Mehrheit der Positionen 1, 3, 5, 7, 9 (11, 13, 15, 17, 19, 21, 23 und 25) und eine ungeladene Aminosäure in mindestens der Mehrheit der Positionen 2, 4, 6, 8, 10 (12, 14, 16, 18, 20, 22, 24) enthält.

25. Knochenmaterial gemäß Anspruch 24, **dadurch gekennzeichnet, daß** die positiv geladenen Aminosäuren aus der Gruppe, bestehend aus Ornithin (O), Lysin (K), Arginin (R) und Histidin (H), ausgewählt sind.

26. Knochenmaterial nach Anspruch 24, **dadurch gekennzeichnet, daß** die ungeladenen Aminosäuren aus der Gruppe, bestehend aus den aliphatischen Aminosäuren Glycin (G), Alanin (A), Valin (V), Leucin (L), Isoleucin (I), den Aminosäuren mit einer dipolaren Seitenkette Methionin (M), Asparagin (N), Glutamin (Q), Serin (S), Threonin (T), den Aminosäuren mit einer aromatischen Seitenkette Phenylalanin (F), Tyrosin (Y), Tryptophan (W), ausgewählt sind.

27. Knochenmaterial gemäß den Ansprüchen 24 bis 26, **dadurch gekennzeichnet, daß** die Mehrheit der positiv geladenen Aminosäuren die Gesamtzahl der positiv geladenen Aminosäuren minus 1 ist.

28. Knochenmaterial gemäß den Ansprüchen 24 bis 27, **dadurch gekennzeichnet, daß** die Mehrheit der ungeladenen Aminosäuren die Gesamtzahl der ungeladenen Aminosäuren minus 1 ist.

29. Knochenmaterial gemäß den Ansprüchen 24 bis 28, **dadurch gekennzeichnet, daß** die Domäne das gesamte Peptid ausmacht.

30. Knochenmaterial gemäß den Ansprüchen 1 bis 29, wobei das N-Ende amidiert ist.

31. Knochenmaterial gemäß den Ansprüchen 1 bis 30, wobei die C-terminale Carbonsäure-Gruppe durch eine Amid-, Ester-, Keton-, Aldehyd- oder Alkohol-Gruppe ersetzt ist.

32. Verfahren zur Herstellung von Knochenmaterial gemäß einem der Ansprüche 1 bis 31, wobei das Knochenmaterial zu Knochenzement gehärtet wird und wobei die AMPs im gehärteten Knochenzement homogen verteilt sind.

33. Verfahren nach Anspruch 32, wobei die AMPs in flüssigem Medium, vorzugsweise Wasser, gelöst werden und mit dem Knochenmaterial nach Härten desselben vermischt werden.

34. Verfahren nach Anspruch 32 oder 33, wobei der gehärtete Knochenzement zu einem Granulat geformt wird.

## Revendications

1. Matériau osseux pour la prévention et le traitement de l'ostéomyélite, lequel matériau est pourvu de peptides antimicrobiens (AMP) constitués d'une chaîne d'acides aminés qui contient un domaine de 10 à 25 acides aminés, où la majorité des acides aminés de l'une des moitiés du domaine sont des acides aminés positivement chargés et la majorité des acides aminés de l'autre moitié du domaine sont des acides aminés non chargés, les AMP pouvant être libérés dans la zone environnante pendant un certain laps de temps, le matériau osseux formant un ciment osseux après durcissement, les AMP étant distribués d'une manière homogène dans le ciment osseux durci.

2. Matériau osseux selon la revendication 1, **caractérisé en ce que** le domaine forme une hélice α, et au moins sur une majorité des positions 1, 2, 5, 6, 9 (12, 13, 16, 19, 20, 23 et 24) contient un acide aminé positivement chargé, sur la position 8 un acide aminé positif ou non chargé, et au moins sur une majorité des positions 3, 4, 7, 10 (11, 14, 15, 17, 18, 21, 22, 25) contient un acide aminé non chargé.

3. Matériau osseux selon la revendication 2, **caractérisé en ce que** les acides aminés positivement chargés sont choisis dans l'ensemble comprenant l'ornithine (O), la lysine (K), l'arginine (R) et l'histidine (H).

4. Matériau osseux selon la revendication 2 ou 3, **caractérisé en ce que** les acides aminés non chargés sont choisis dans l'ensemble comprenant les acides aminés aliphatiques glycine (G), alanine (A), valine (V), leucine (L), isoleucine (I), les acides aminés ayant une chaîne latérale dipolaire méthionine (M), asparagine (N), glutamine (Q), sérine (S), thréonine (T), les acides aminés ayant une chaîne latérale aromatique phénylalanine (F), tyrosine (Y), tryptophane (W).

5. Matériau osseux selon les revendications 2 à 4, **caractérisé en ce que** la majorité des acides aminés positivement chargés aient le nombre total des acides aminés positivement chargés, diminués de 1.

6. Matériau osseux selon les revendications 2 à 5, **caractérisé en ce que** la majorité des acides aminés non chargés aient le nombre total des acides aminés non chargés, diminués de 1.

7. Matériau osseux selon les revendications 2 à 6, **caractérisé en ce que** le domaine constitue la totalité du peptide.

8. Matériau osseux selon les revendications 2 à 7, dont le domaine a la séquence d'acides aminés suivante :

KRLFKELKFSLRKY (peptide 3).

9. Matériau osseux selon les revendications 2 à 7, dont le domaine a la séquence d'acides aminés suivante :

KRLFKELLLFSLRKY (peptide 4).

10. Matériau osseux selon les revendications 2 à 7, dont le domaine a la séquence d'acides aminés suivante :

KRLFKELKKSLRKY (peptide 5).

11. Matériau osseux selon les revendications 2 à 7, dont le domaine a la séquence d'acides aminés suivante :

KRLFKELLLKSLRKY (peptide 6).

12. Matériau osseux selon les revendications 2 à 7, dont le domaine a la séquence d'acides aminés suivante :

OOLFOELOOSLOOY (peptide 7).

13. Matériau osseux selon les revendications 2 à 7, dont le domaine a la séquence d'acides aminés suivante :

OOLFOELLOSLOOY (peptide 8).

14. Matériau osseux selon les revendications 2 à 7, dont le domaine a la séquence d'acides aminés suivante :

KRLFKKLKFSLRKY (peptide 9).

15. Matériau osseux selon les revendications 2 à 7, dont le domaine a la séquence d'acides aminés suivante :

KRLFKKLLFSLRKY (peptide 10).

16. Matériau osseux selon la revendication 1, **caractérisé en ce que** le domaine forme une hélice α, et au moins sur une majorité des positions 1 à 6 (ou 7 ou 8 ou 9 ou 10 ou 11 ou 12) contient un acide aminé non chargé et sur les positions 7 (ou 8 ou 9 ou 10 ou 11 ou 12 ou 13) à 25 un acide aminé positivement chargé.

17. Matériau osseux selon la revendication 1, **caractérisé en ce que** le domaine forme une hélice α, et sur une majorité des positions 1 à 6 (ou 7 ou 8 ou 9 ou 10 ou 11 ou 12) contient un acide aminé positivement chargé et sur les positions 7 (ou 8 ou 9 ou 10 ou 11 ou 12 ou 13) à 25 un acide aminé non chargé.

18. Matériau osseux selon la revendication dans la revendication 16 ou 17, **caractérisé en ce que** les acides aminés positivement chargés sont choisis dans l'ensemble comprenant l'ornithine (O), la lysine (K), l'arginine (R) et l'histidine (H).

19. Matériau osseux selon la revendication 16, 17 ou 18, **caractérisé en ce que** les acides aminés non chargés sont choisis dans l'ensemble comprenant les acides aminés aliphatiques glycine (G), alanine (A), valine (V), leucine (L), isoleucine (I), les acides aminés ayant une chaîne latérale dipolaire méthionine (M), asparagine (N), glutamine (Q), sérine (S), thréonine (T), les acides aminés ayant une chaîne latérale aromatique phénylalanine (F), tyrosine (Y), tryptophane (W).

20. Matériau osseux selon les revendications 16 à 19, **caractérisé en ce que** les acides aminés positivement chargés aient le nombre total des acides aminés positivement chargés, diminués de 1.

21. Matériau osseux selon les revendications 16 à 20, **caractérisé en ce que** la majorité des acides aminés non chargés aient le nombre total des acides aminés non chargés, diminués de 1.

22. Matériau osseux selon les revendications 16 à 21, **caractérisé en ce que** le domaine constitue la totalité du peptide.

23. Matériau osseux selon les revendications 16 et 18 à 22, dont le domaine a la séquence d'acides aminés suivante:

LLLFLLKKRKKRKY (peptide 11)

24. Matériau osseux selon la revendication 1, **caractérisé en ce que** le domaine forme ce que l'on appelle un brin β et contient un acide aminé positivement chargé sur au moins une majorité des positions 1, 3, 5, 7, 9 (11, 13, 15, 17, 19, 21, 23 et 25) et un acide aminé non chargé sur au moins une majorité des positions 2, 4, 6, 8, 10 (12, 14, 16, 18, 20, 22, 24).

25. Matériau osseux selon la revendication 24, **caractérisé en ce que** les acides aminés positivement chargés sont choisis dans l'ensemble comprenant l'ornithine (O), la lysine (K), l'arginine (R) et l'histidine (H).

26. Matériau osseux selon la revendication 24, **caractérisé en ce que** les acides aminés non chargés sont choisis dans l'ensemble comprenant les acides aminés aliphatiques glycine (G), alanine (A), valine (V), leucine (L), isoleucine (I), les acides aminés ayant une chaîne latérale dipolaire méthionine (M), asparagine (N), glutamine (Q), sérine (S), thréonine (T), les acides aminés ayant une chaîne latérale aromatique phénylalanine (F), tyrosine (Y), tryptophane (W).

27. Matériau osseux selon les revendications 24 à 26, **caractérisé en ce que** les acides aminés positivement chargés aient le nombre total des acides aminés positivement chargés, diminués de 1.

28. Matériau osseux selon les revendications 24 à 27, **caractérisé en ce que** la majorité des acides aminés non chargés aient le nombre total des acides aminés non chargés, diminués de 1.

29. Matériau osseux selon les revendications 24 à 28, **caractérisé en ce que** le domaine constitue la totalité du peptide.

30. Matériau osseux selon les revendications 1 à 29, dans lequel le site N-terminal est amidé.

31. Matériau osseux selon les revendications 1 à 30, dans lequel le groupe acide carboxylique C-terminal est remplacé par un groupe amide, ester, cétone, aldéhyde ou alcool.

32. Procédé de fabrication d'un matériau osseux selon l'une quelconque des revendications 1 à 31, dans lequel le matériau osseux est durci pour donner un ciment osseux, et les AMP sont distribués d'une manière homogène dans le ciment osseux durci.

33. Procédé selon la revendication 32, dans lequel les AMP sont dissous dans un milieu liquide, de préférence de l'eau, et sont mélangés au matériau osseux après son durcissement.

34. Procédé selon la revendication 32 ou 33, dans lequel le ciment osseux durci est façonné pour donner un granulé.
